# EUROPÄISCHE PATENTANMELDUNG

(11) **EP 1 163 952 A1**
(43) Veröffentlichungstag der Anmeldung: **19.12.2001**
(21) Anmeldenummer: 00810512.4
(22) Anmeldetag: 14.06.2000
(51) Int. Cl.: B01J 8/02, C07C 5/48

(54) **Mikrokanäle enthaltendes Festbett angeordnet in einem rohrförmigen Reaktorteil**

(71) Anmelder: Sulzer Chemtech AG, 8404 Winterthur (CH); Ecole Polytechnique Fédérale de Lausanne (EPFL), 1015 Lausanne (CH)
(72) Erfinder: Wolfrath, Olivier, 1162 St. Prex (CH); Kiwi-Minsker, Lioubov, 1026 Echandens (CH); Renken, Albert, 1025 St. Sulpice (CH)
(74) Vertreter: Ehrsam, Christian

(57) **Zusammenfassung**

Das Festbett (1a,1b), insbesondere ein Festbett mit Katalysator, ist anisotrop strukturiert und in einem rohrförmigen Teil (21) eines Reaktors (20) angeordnet. Dieser Reaktorteil (21) ist in Richtung seiner Längserstreckung von einem Reaktionsfluid (30,40) durchströmbar. In einem das Festbett enthaltenden Bereich weist er Querschnittsflächen auf, deren Flächeninhalte konstant sind. Diese Querschnittsflächen haben mindestens in einer Richtung Wandabstände (d,a), die wesentlich kleiner als die Längserstreckung des Reaktorteils sind. Die Festbettstruktur umfasst eine Vielzahl von Mikrokanälen in Strömungsrichtung, die weitgehend homogen über die Querschnittsfläche verteilt sind. Die hydraulischen Durchmesser der Mikrokanäle sind kleiner als 1mm, so dass bei der Durchführung einer Reaktion das zu behandelnde Reaktionsfluid die Festbettstruktur laminar durchströmt.
Vorzugsweise besteht die Festbettstruktur aus einem Strang oder dichten Bündel von in der Strömungsrichtung angeordneten Fäden (10).

## Beschreibung

Die Erfindung betrifft ein Festbett, insbesondere ein Festbett mit Katalysator, das in einem rohrförmigen Reaktorteil angeordnet ist. Dieser Reaktorteil ist in Richtung seiner Längserstreckung von einem Reaktionsfluid, insbesondere einem gasförmigen Reaktionsfluid durchströmbar und weist längs einem das Festbett enthaltenden Bereich eine konstant bleibende Querschnittsfläche auf. Diese Querschnittsfläche, die kreis- oder ringförmig oder auch von anderer Form sein kann, hat mindestens in einer Richtung Wandabstände, die wesentlich kleiner als die Längserstreckung sind. Die Erfindung bezieht sich auch auf Reaktoren, jeweils mit mindestens einem rohrförmigen Reaktorteil, der ein erfindungsgemässes Festbett enthält.

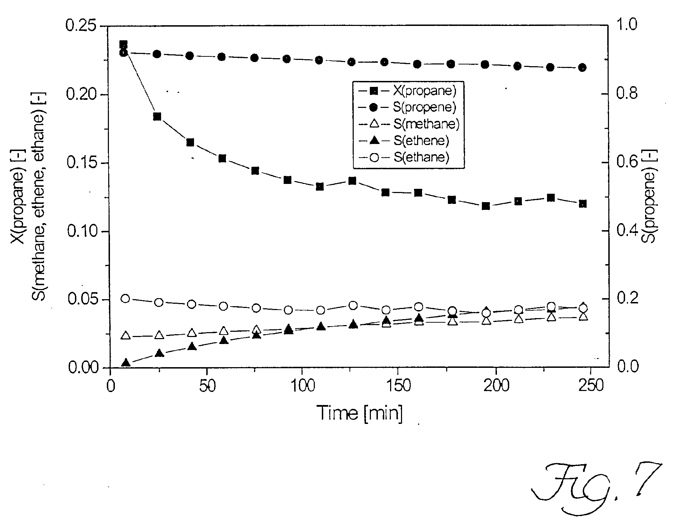

Ausgangspunkt der erfindungsgemässen Lehre ist ein katalytisches Verfahren zur Dehydrierung von Propan zu Propylen. Mit einem Platin-Zinn-Katalysator (0.5%Pt / 1%Sn / ASF, ASF = "Alumina-Silica Fibrous support") in Form eines faserigen Festbetts lässt sich bei 823 K eine Dehydrierung durchführen, bei der im thermodynamischen Gleichgewicht 24% des Propans C₃H₈ zu Propylen C₃H₆ umgewandelt ist (siehe Fig. 7). Als Nebenprodukte entstehen Methan, Ethan und Ethylen. Ausserdem gibt es eine Kohlenstoffabscheidung auf dem Festbett, die ein periodisches Regenerieren des Katalysators erforderlich macht. Indem man den abgespaltenen Wasserstoff aus dem Prozess entfernt, kann die Umwandlung des Propans in Propylen um rund das Dreifache vergrössert werden. Die Wasserstoffentfernung lässt sich mittels einer Gaspermeation in einem Reaktorrohr durch eine semipermeable Rohrwand (Pd/Ag-Rohr, Wanddicke 70 µm, Rohrdurchmesser 6mm) realisieren. Damit die Konzentration des Wasserstoffs im Reaktorrohr genügend tief abgesenkt werden kann, muss der Rohrdurchmesser klein sein.

Bei einer sehr vorteilhaften Durchführung der Dehydrierung ist der Reaktor so ausgebildet, dass er durch die semipermeable Wand in zwei Zonen unterteilt ist. In der einen Zone wird die Dehydrierung durchgeführt. In der anderen Zone wird der entfernte Wasserstoff in einem zweiten Gasstrom, der Sauerstoff enthält, zu Wasser verbrannt. Gleichzeitig wird eine Kohlenstoffabscheidung beseitigt und damit der Katalysator regeneriert. Die bei diesen zwei exothermen Reaktionen frei werdende Wärme gelangt durch Wärmeübertragung in die erste Zone, wo sie bei der endothermen Reaktion der Dehydrierung wieder genutzt wird. Die zweite Zone hat also funktionell eine dreifache Bedeutung: als Wasserstoffsenke, als Wärmequelle und als Ort der Regenerierung des Katalysators. Die endotherme Reaktion auf der einen Seite und die exotherme Reaktion auf der anderen Seite werden periodisch im zeitlichen Wechsel in den beiden Reaktorteilen durchgeführt.

Aufgabe der Erfindung ist es, ein Festbett für ein enges Rohr zu schaffen, das sich als Katalysatorträger für das oben beschriebene Dehydrierungsverfahren eignet, das durch ein periodisches Regenerieren des Katalysators, eine günstige Beeinflussung der erwünschten Umwandlung sowie einen vorteilhaften Wärmehaushalt charakterisiert ist. Diese Aufgabe wird durch das im Anspruch 1 definierte Festbett gelöst. Das erfindungsgemässe Festbett ermöglicht es, enge Reaktorteile, die rohrförmig sind und die konstante Querschnittsflächen aufweisen, weitgehend homogen zu füllen. Ein Reaktionsfluid durchströmt das Festbett laminar, so dass vorteilhafterweise sich enge Verweilzeitverteilungen für die zu behandelnden Komponenten des Reaktionsfluides ergeben. Verglichen mit Strömungen durch Pulverschüttungen ist die Strömung durch das erfindungsgemässe Festbett mit einem kleineren Druckabfall verbunden.

Das Festbett, insbesondere ein Festbett mit Katalysator, ist anisotrop strukturiert und in einem rohrförmigen Teil eines Reaktors angeordnet. Dieser Reaktorteil ist in Richtung seiner Längserstreckung von einem Reaktionsfluid durchströmbar. In einem das Festbett enthaltenden Bereich weist er Querschnittsflächen auf, deren Flächeninhalte konstant sind. Diese Querschnittsflächen haben mindestens in einer Richtung Wandabstände, die wesentlich kleiner als die Längserstreckung des Reaktorteils sind. Die Festbettstruktur umfasst eine Vielzahl von in Strömungsrichtung verlaufenden Mikrokanälen, die weitgehend homogen über die Querschnittsfläche verteilt sind. Die hydraulischen Radien der Mikrokanäle sind kleiner als 0.5 mm, so dass bei der Durchführung einer Reaktion das zu behandelnde Reaktionsfluid die Festbettstruktur laminar durchströmt (wobei der hydraulische Radius definiert ist als Quotient von Flächeninhalt der durchströmten Querschnittsfläche und benetztem Umfang).

Die abhängigen Ansprüche 2 bis 5 betreffen vorteilhafte Ausführungsformen des erfindungsgemässen Festbetts. Die Ansprüche 6 bis 9 beziehen sich auf Reaktoren, die das erfindungsgemässe Festbett enthalten. Eine Verwendungsmöglichkeit ist Gegenstand des Anspruchs 10.

Nachfolgend wird die Erfindung anhand der Zeichnungen erläutert. Es zeigen:
- Fig. 1: eine Anlage, mit der sich beispielsweise eine Dehydrierung von Propan zu Propylen durchführen lässt,
- Fig. 2: ein Bündel von Fäden eines erfindungsgemässen Festbetts,
- Fig. 3: eine zweite Form eines Fadens für ein erfindungsgemässes Festbett,
- Fig. 4, 5: schematische Darstellungen von Querschnitten durch erfindungsgemässe Reaktoren, die jeweils eine Mehrzahl von rohrförmigen Reaktorteilen und einen komplementären Reaktorteil umfassen,
- Fig. 6: einen Sandwichaufbau eines erfindungsgemässen Reaktors mit Kreuzstromführung der Reaktionsfluide und
- Fig. 7: ein Diagramm mit Messresultaten für die beschriebene Dehydrierung von Propan.

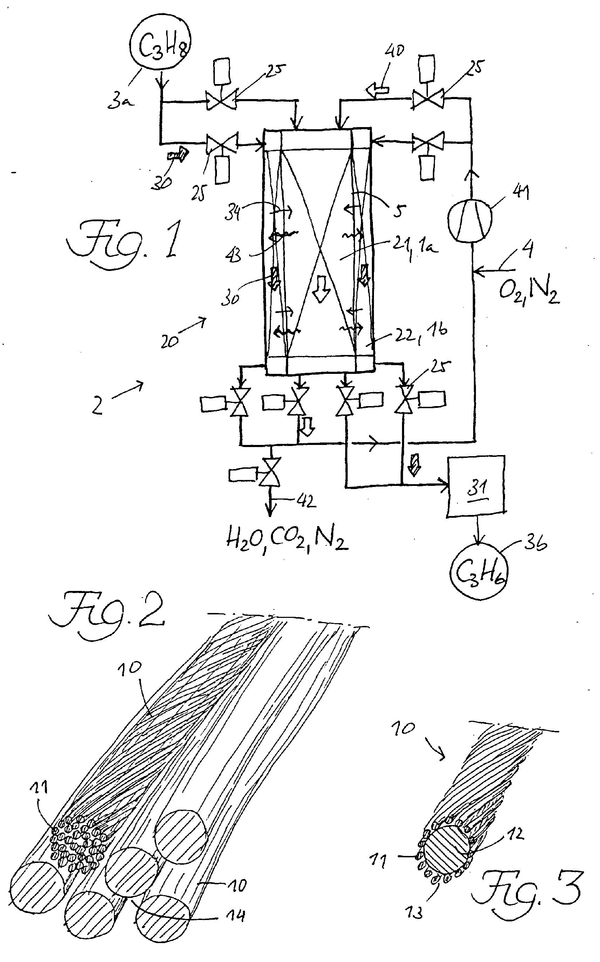

In einer Anlage 2 mit einem Reaktor 20, die in Fig. 1 scgematisch dargestellt ist, lässt sich ein Reaktionsfluid 3a, dessen Transportweg mit schraffierten Pfeilen angedeutet ist, mittels einer endothermen Reaktion zu einem Produkt 3b umwandeln, wobei eine Komponente 34 freigesetzt wird. Beispielsweise wird Propan 3a zu Propylen 3b dehydriert, wobei gasförmiger Wasserstoff 34 entsteht. Die endotherme Reaktion kann in Festbetten 1 a oder 1 b stattfinden; sie erfolgt bei der in Fig. 1 dargestellten Prozessphase im Festbett 1b. Ein zweites Reaktionsfluid 4 (Pfeile 40) wird für die Durchführung von mindestens einer exothermen Reaktion in dem anderen Festbett 1 a verwendet. Der Reaktor 20 ist durch eine semipermeable Wand 5 in zwei Reaktorteile 21 und 22 unterteilt. Die freigesetzte Komponente 34 diffundiert durch die Wand 5 und bildet ein Edukt der exothermen Reaktion. Die freigesetzte Wärme 43 wird in der umgekehrten Richtung in die endotherme Reaktion übertragen. Ein periodischer Wechsel der Prozessphasen wird mittels Umschalten von steuerbaren Schliessorganen 25 herbeigeführt.

Das zweite Reaktionsfluid 4, 40 ist ein Inertgas, das Sauerstoff (5% bei der Propanhydrierung) enthält und das im vorliegenden Beispiel mittels eines Gebläses 41 in einem Kreislauf durch den Reaktor 20 getrieben wird. Der bei der exothermen Reaktion verbrauchte Sauerstoff kann in Form von Luft 4 (Stickstoff N₂ und Sauerstoff O₂) aus der Umgebung nachgespeist werden. Ein Gasüberschuss 42, der das bei der exothermen Reaktion gebildete Wasser und Kohlendioxid (H₂O, CO₂ ) enthält, kann an die Umgebung abgeführt werden. Das Produkt 3b wird in einer Teilanlage 31 von nicht reagiertem Edukt 3a und den Nebenprodukten getrennt.

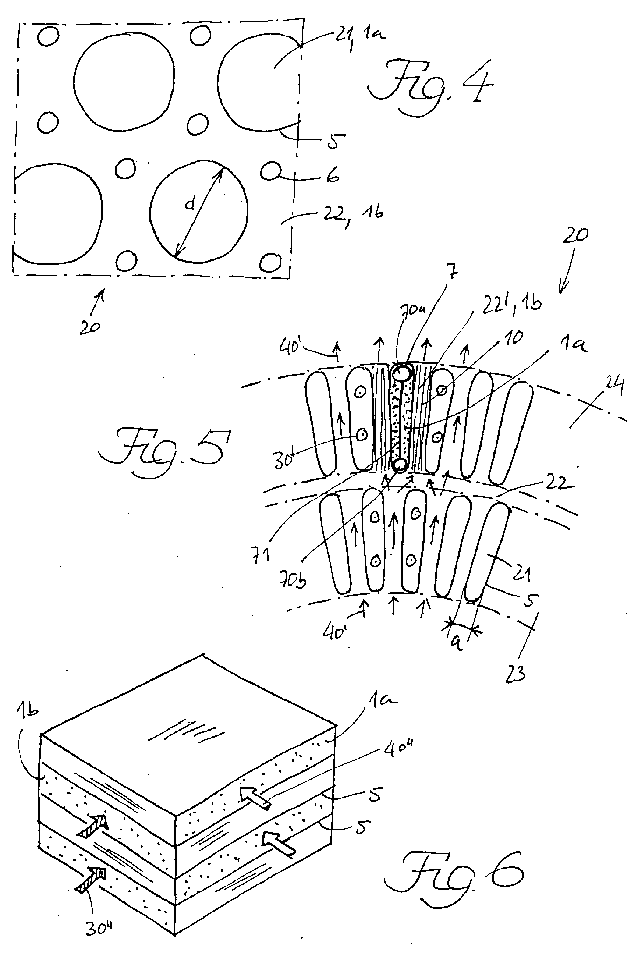

Fig. 2 zeigt ein Bündel von Fäden 10 eines erfindungsgemässen Festbetts 1a oder 1b. Eine zweite Form eines Fadens 10 ist in Fig. 3 dargestellt. Die Fäden 10 sind aus Fasern oder Filamenten 11 zusammengesetzt, deren Durchmesser zumindest zu einem Teil Werte im Bereich von 3 bis 10 µm haben. Die Fasern oder Filamente 11 können auf ihrer Oberfläche, die vorzugsweise porös ausgebildet ist, den Katalysator tragen. In Fig. 2 ist nur einer der Fäden 10 mit einem faserigen Aufbau dargestellt. Es sind aber alle Fäden 10 gleich ausgebildet. Sie sind jeweils aus einer Vielzahl (Grössenordnung 100) von zusammengezwirnten Filamenten 11 gebildet. Im rohrförmigen Reaktorteil 21 eingebaut sind die Fäden 10 dichter angeordnet als in Fig. 2 dargestellt. In einer Anordnung von maximaler Dichte haben die hydraulischen Durchmesser (= zweimal hydraulischer Radius) der zwischen den Fäden 10 liegenden Mikrokanäle 14 einen minimalen Wert.

Der Faden 10 in Fig. 3 hat einen homogenen Kern 12, der von einem den Katalysator tragenden Randbereich 13 ummantelt ist. Der Katalysatorträger des Randbereichs 13 ist einlagig aus Fasern 11 zusammengesetzt; er kann auch mehrlagig ausgebildet sein (nicht dargestellt). Auch die Filamente 11 können jeweils aus einem homogenen Kern und einem den Kern ummantelnden, den Katalysator tragenden Randbereich aufgebaut sein. Die Kerne 12 der Fäden 10 und auch die Filamentkerne können beispielsweise aus Glas, insbesondere Quarzglas, Kohlenstoff oder einer metallischen Legierung bestehen. Der Filamentmantel kann ein poröses, keramisches Material, beispielsweise Aluminiumoxid sein.

Die Figuren 4 und 5 zeigen in schematischen Darstellungen Querschnitte durch erfindungsgemässe Reaktoren, die jeweils eine Mehrzahl von rohrförmigen Reaktorteilen 21 und einen komplementären Reaktorteil 22 umfassen. Die zylinder- oder taschenförmigen Reaktorteile 21 sind durch semipermeable Wände 5 von dem komplementären Reaktorteil 22 getrennt. In den rohrförmigen Reaktorteilen 21 sind jeweils die gleiche Reaktion vorgesehen - entweder die endo- oder exotherme - und im komplementären Reaktorteil 22 die andere Reaktion - die exo- bzw. endotherme. Wie schon im Beispiel der Fig. 1 werden die beiden Reaktionen periodisch im zeitlichen Wechsel in den rohrförmigen Reaktorteilen 21 bzw. im komplementären Reaktorteil 22 durchgeführt. Die rohrförmigen Reaktorteile 21 enthalten Festbette 1a, die eine faserige Trägerstruktur aufweisen. Der komplementäre Reaktorteil 22 enthält ein Festbett 1b, das ebenfalls eine faserige Trägerstruktur haben kann, das aber auch aus einer Schüttung von granulat- oder pulverförmigem Material bestehen kann.

Die Festbette 1a, 1b aller Reaktorteile 21, 22 weisen mit Vorteil alle eine faserige Trägerstruktur auf. Dabei kann die Faserrichtung der Festbette 1a, 1b in allen Reaktorteilen 21, 22 gleich orientiert sein. Es können aber - siehe Fig. 5 - die Faserrichtungen der Festbette 1a in den rohrförmigen Reaktorteilen 21 auch quer zu der Faserrichtung des komplementären Reaktorteils 22 orientiert sein.

Der Reaktor 20 nach Fig. 5 hat eine zentralsymmetrische Architektur; er umfasst mehrere Ringe 23, 24, die alternierend durch a) rohrförmige, axial ausgerichtete Reaktorteile 21 und b) Kanäle 22' des komplementären Reaktorteils 22 aufgebaut sind. In den Kanälen 22' sind die Fäden 10 des Festbetts 1b radial ausgerichtet. Die Querschnittsflächen der Reaktorteile 21 sind keilförmig, so dass die radialen Wände der Kanäle 22' weitgehend parallel zueinander sind. Ein erstes Reaktionsfluid 30' strömt in axialer Richtung; ein zweites Reaktionsfluid 40' strömt radial von innen nach aussen (eine Strömung nach innen wäre selbstverständlich auch möglich). Die Reaktorteile 21 enthalten Träger 7 (Rohre 70a, 70b und Verbindungslamellen 71), auf denen die folienartigen Wände 5 aufgespannt sind.

Für den Reaktor der Fig. 4 sind weitere Konstruktionselemente 6 angedeutet. Diese Elemente 6 können Füllkörper, Stützelemente oder Rohre eines Wärmetauschers sein. Ein Wärmetauscher kann notwendig sein, um überschüssige Wärme aus dem Reaktor abzuführen. Die Rohre 70a, 70b des Reaktors in Fig. 5 können ebenfalls als Wärmetauscherelemente vorgesehen sein. Die Konstruktionselemente 6 können auch andere Formen als in Fig. 4 dargestellt haben.

Die Innenräume der rohrförmigen Reaktorteile 21 sind eng: d. h. die Querschnittsflächen haben mindestens in einer Richtung Wandabstände - Durchmesser d (siehe Fig. 4) oder Breiten a (siehe Fig. 5) - die wesentlich kleiner als die Längserstreckung des Reaktorteils 21 sind. Diese Wandabstände d oder a haben Werte im Bereich von 1 bis 20 mm, vorzugsweise 3 bis 10 mm. Um eine homogene Verteilung der Mikrokanäle 14 (siehe Fig. 2) über die Querschnittsfläche erhalten zu können, müssen die Fäden 10 ebenfalls homogen verteilt sein und ihre Durchmesser zumindest zu einem Teil Werte im Bereich von 0.1 bis 1 mm haben. Bei einer solchen Anordnung der Fäden 10 haben die zwischen ihnen liegenden Mikrokanäle kleine hydraulische Durchmesser, deren Werte kleiner als 1 mm sind. Folglich durchströmt das zu behandelnde Reaktionsfluid die Festbettstruktur in laminarer Form. Ein Druckabfall ist gering.

Fig. 6 zeigt sehr schematisch einen Sandwichaufbau eines erfindungsgemässen Reaktors, in dem die Reaktionsfluide 30" und 40" als sich kreuzende Ströme geführt werden. Ein solcher Reaktor hat einen ähnlichen Aufbau wie jener der Fig. 5.

Fig. 7 zeigt ein Diagramm mit Messresultaten für die beschriebene Dehydrierung von Propan (4.9 s Verweilzeit im Katalysator, 823 K, 1.4 bar, 7.5 Nml/min Propan am Rohreingang, 6 mm Rohrdurchmesser). Die relative Menge des umgesetzten Propans ist mit X bezeichnet; sie beträgt ungefähr 23% für den frisch regenerierten Katalysator und fällt nach einer Versuchsdauer von 4 Stunden auf rund 13% ab. Die Selektivität S der Propylenerzeugung ist rund 90%; sie verändert sich nur wenig. Die Selektivität S für die Nebenprodukte ist relativ gering, kleiner als 0.05%.

Das erfindungsgemässe Festbett lässt sich auch mit Vorteil für nichtkatalytische Reaktionen verwenden, in denen eine enge Verweilzeitverteilung erforderlich ist, um beispielsweise unerwünschte, bei Folgereaktionen auftretende Nebenprodukte zu vermeiden. Ein weiteres Beispiel ist eine heterogene Reaktion zwischen nicht mischbaren Fuiden, beispielsweise einem Gas und einer Flüssigkeit; bei Durchströmen eines Gemenges solcher Fluide lässt sich im erfindungsgemässen Festbett ein grosse Grenzfläche zwischen den Fluiden aufrechterhalten.

Vorteilhaft ist auch ein Reaktor mit erfindungsgemässen Festbetten, wenn -zwei Festbette enthaltende Reaktorteile durch eine Wand getrennt sind und eine endotherme Reaktion im einen Reaktorteil mit einer exothermen Reaktion im anderen Reaktorteil lediglich thermisch gekoppelt werden.

## Patentansprüche

1. Festbett (1a, 1b), insbesondere ein Festbett mit Katalysator, das anisotrop strukturiert ist und das in einem rohrförmigen Teil (21) eines Reaktors (20) angeordnet ist, wobei dieser Reaktorteil (21), der in Richtung seiner Längserstreckung von einem Reaktionsfluid (30, 40) durchströmbar ist, in einem das Festbett enthaltenden Bereich Querschnittsflächen aufweist, deren Flächeninhalte konstant sind, diese Querschnittsflächen mindestens in einer Richtung Wandabstände (d, a) haben, die wesentlich kleiner als die Längserstreckung des Reaktorteils sind, und die Festbettstruktur eine Vielzahl von Mikrokanälen (14) in Strömungsrichtung umfasst, die weitgehend homogen über die Querschnittsfläche verteilt sind und die hydraulische Durchmesser kleiner als 1mm haben, so dass bei der Durchführung einer Reaktion das zu behandelnde Reaktionsfluid die Festbettstruktur laminar durchströmt.

2. Festbett nach Anspruch 2, **dadurch gekennzeichnet, dass** die genannten Wandabstände (d, a) Werte im Bereich von 1 bis 20 mm, vorzugsweise 3 bis 10 mm haben.

3. Festbett nach Anspruch 1 oder 2, **dadurch gekennzeichnet, dass** die Festbettstruktur aus einem Strang oder dichten Bündel von in der Strömungsrichtung angeordneten Fäden (10) besteht, die Fäden insbesondere eine Trägerstruktur für einen Katalysator bilden, und die Fäden (10) aus Fasern oder Filamenten (11) zusammengesetzt sind, deren Durchmesser zumindest zu einem Teil Werte im Bereich von 3 bis 10 µm haben.

4. Festbett nach Anspruch 3, **dadurch gekennzeichnet, dass** die Durchmesser der Fäden (10) zumindest zu einem Teil Werte im Bereich von 0.1 bis 1 mm haben und dass vorzugsweise alle Fäden (10) gleich ausgebildet sind, wobei sie insbesondere jeweils aus einer Vielzahl von zusammengezwirnten Filamenten (11) bestehen.

5. Festbett nach Anspruch 4, **dadurch gekennzeichnet, dass** ein Katalysator auf der Festbettstruktur aufgebracht ist und die Fäden (10) und/oder Filamente (11) jeweils aus einem homogenen Kern (12) und einem den Kern ummantelnden, den Katalysator tragenden Randbereich (13) aufgebaut sind, wobei beispielsweise der Kern aus Glas, insbesondere Quarzglas, Kohlenstoff oder einer metallischen Legierung besteht und der Katalysatorträger des Randbereichs aus Fasern (11) zusammengesetzt ist oder ein poröses, keramisches Material, beispielsweise Aluminiumoxid ist.

6. Reaktor (20) mit Festbett (1a, 1b) gemäss einem der Ansprüche 1 bis 5, **dadurch gekennzeichnet, dass** ein rohrförmiger Reaktorteil (21) von einem zweiten Reaktorteil (22) durch eine Wand (5) getrennt ist, dass im Festbett (1a, 1b) des einen Reaktorteils (21, 22) eine endotherme Reaktion und im Festbett (1b, 1a) des anderen Reaktorteils (22, 21) eine exotherme Reaktion durchführbar ist, und dass beim Betreiben des Reaktors die endotherme Reaktion thermisch mit der exothermen Reaktion gekoppelt ist.

7. Reaktor (20) mit Festbett (1a, 1b) gemäss einem der Ansprüche 1 bis 5, **dadurch gekennzeichnet, dass** ein rohrförmiger Reaktorteil (21) von einem zweiten Reaktorteil (22) durch eine semipermeable Wand (5) getrennt ist, dass im Festbett (1a, 1b) eine endotherme Reaktion durchführbar ist, bei der aus einem ersten Reaktionsfluid (3a, 30) eine Komponente abspaltbar ist, insbesondere katalytisch abspaltbar ist, für welche die semipermeable Wand durchlässig ist, und dass beim Betreiben des Reaktors die endotherme Reaktion in dem einen Reaktorteil (22) durchgeführt wird, während mindestens eine exotherme Reaktion im anderen Reaktorteil (21) durchgeführt wird, bei der die abgespaltene, durch die Wand diffundierte Komponente (43) mit einem zweiten Reaktionsfluid (4, 40) reagiert, wobei die exotherme Reaktion zumindest einen Teil (34) der für die endotherme Reaktion benötigte Wärme liefert, und das thermodynamische Gleichgewicht der endothermen Reaktion durch Entfernen der abgespaltenen Komponente zugunsten eines erwünschten Produkts (3b) beeinflusst wird.

8. Reaktor nach Anspruch 6 oder 7, **dadurch gekennzeichnet, dass** ein oder mehrere rohrförmige, insbesondere zylinder- oder taschenförmige Reaktorteile (21) durch Wände (5) von einem komplementären Reaktorteil (22) getrennt sind, dass in den rohrförmigen Reaktorteilen jeweils die gleiche Reaktion - entweder die endo- oder exotherme - sowie im komplementären Reaktorteil die andere Reaktion - die exo-bzw. endotherme - vorgesehen sind und dass ausserdem vorgesehen ist, die beiden Reaktionen periodisch im zeitlichen Wechsel in den rohrförmigen Reaktorteilen bzw. im komplementären Reaktorteil durchzuführen.

9. Reaktor nach Anspruch 8, **dadurch gekennzeichnet, dass** die Festbette (1a, 1b) aller Reaktorteile (21, 22) eine faserige Struktur aufweisen, wobei die Faserrichtungen der Festbette in allen Reaktorteilen gleich orientiert sind, oder die Faserrichtungen der Festbette in den rohrförmigen Reaktorteilen (21) quer zu der Faserrichtung des komplementären Reaktorteils (22) orientiert sind.

10. Verwendung eines Reaktors gemäss Anspruch 7 und Anspruch 8 oder 9, **dadurch gekennzeichnet, dass** das erste Reaktionsfluid (3a) ein Kohlenwasserstoff, insbesondere Propan ist, mit dem katalytisch eine Dehydrierung unter einer selektiven Bildung eines Produkts (3b), insbesondere von Propylen durchgeführt wird, dass das zweite Reaktionsfluid (4, 40) ein Sauerstoff enthaltendes Medium, insbesondere ein Inertgas ist, mit dem ein bei der Dehydrierung freigesetzter Wasserstoff (34) zu Wasser oxidiert wird, und dass mit dem zweiten Reaktionsfluid noch eine weitere exotherme Reaktion durchgeführt wird, bei welcher eine bei der Dehydrierung auf dem Festbett entstandene Kohlenstoffabscheidung beseitigt und damit der Katalysator regeneriert wird.
